# EUROPEAN PATENT APPLICATION

(11) **EP 4 726 726 A1**
(43) Date of publication of application: **15.04.2026**
(21) Application number: 25205340.0
(22) Date of filing: 29.09.2025
(51) Int. Cl.: G16H 10/60, G16H 15/00, G16H 80/00

(54) **A HEALTHCARE PROVIDER ASSISTANT SYSTEM AND A COMPUTER-IMPLEMENTED METHOD**

(30) Priority: 08.10.2024 GB 202414773
(71) Applicant: Vodafone Group Services Limited, Newbury RG14 2FN (GB)
(72) Inventor: MEY, Oliver, London, W2 6BY (GB); KUSA, Natalia, London, W2 6BY (GB); PETZOLD, Thomas, London, W2 6BY (GB)
(74) Representative: Boult Wade Tennant LLP

(57) **Abstract**

Aspects of the present invention relate to a healthcare provider assistant system, A computer-implemented method for assisting a healthcare provider and an emergency vehicle comprising the healthcare provider assistant system. The healthcare provider assistant system comprising: one or more processors collectively configured to: receive voice data from one or more microphones; recognise a first voice as a healthcare provider voice previously registered with the healthcare provider assistant system; assign a second voice as a patient voice; generate a transcription of the voice data, wherein the transcription identifies words spoken by the healthcare provider voice and words spoken by the patient voice; provide the transcription and/or the voice data to a trained language model; provide a set of prompts to the trained language model; wherein the set of prompts comprise: a first subset of prompts associated with the healthcare provider voice; wherein each prompt comprises one or more tasks for the trained language model to complete, using the transcription and/or voice data; wherein at least one prompt of the first subset of prompts relates to obtaining healthcare data based on words spoken by the healthcare provider voice; wherein the trained language model is arranged to process the transcription and/or the voice data and the set of prompts to provide responses to one or more prompts from the set of prompts.

## Description

The present disclose relates to assisting a healthcare provider, with consultations, examinations and treatments of patients. Aspects of the invention relate to a healthcare provider assistant system and a computer-implemented method.

### BACKGROUND

Interactions between patients and healthcare providers are often documented. For example, a patient's visit to a healthcare provider, such as a doctor, may involve a consultation, examination or treatment, which subsequently has a report written which may include a summary of what took place, as well as subsequent actions recommended or prescribed treatments/drugs. For example, the report may include a referral and/or prescription given. Examples also include radiology reports, discharge summaries and patient clinic letters. Preparing these reports is time-consuming for healthcare providers and may also be incomplete or inaccurate due to their time-consuming nature and being completed after the patient's visit has concluded. According to studies, doctors in hospitals can spend around three hours per day on documentation, causing significant inefficiency in healthcare.

### BRIEF SUMMARY OF THE DISCLOSURE

Aspects and embodiments of the invention provide a healthcare provider assistant system, A computer-implemented method for assisting a healthcare provider and an emergency vehicle comprising the healthcare provider assistant system as claimed in the appended claims.

According to an aspect of the present invention there is provided a healthcare provider assistant system comprising: one or more processors collectively configured to:
receive voice data from one or more microphones; recognise a first voice as a healthcare provider voice previously registered with the healthcare provider assistant system;
assign a second voice as a patient voice; generate a transcription of the voice data, wherein the transcription identifies words spoken by the healthcare provider voice and words spoken by the patient voice; provide the transcription and/or the voice data to a trained language model; provide a set of prompts to the trained language model; wherein the set of prompts comprise: a first subset of prompts associated with the healthcare provider voice; wherein each prompt comprises one or
more tasks for the trained language model to complete, using the transcription and/or voice data;
wherein at least one prompt of the first subset of prompts relates to obtaining healthcare data based on words spoken by the healthcare provider voice; wherein the trained language model is arranged to process the transcription and/or the voice data and the set of prompts to provide responses to one or more prompts from the set of prompts.

Optionally, the set of prompts additionally comprise a second subset of prompts associated with the patient voice.

Optionally, at least one prompt of the second subset of prompts relates to obtaining healthcare data based on words spoken by the patient voice.

Optionally, the healthcare provider assistant system additionally comprises a display, wherein the display is configured to display at least one of: information and generated content resulting from responses to one or more prompts from the set of prompts.

Optionally, the healthcare provider assistant system is further configured to display the information relating to the responses to one or more prompts on the display whilst receiving additional voice data from the one or more microphones and continuing to generate the transcription of the voice data.

Optionally, the healthcare provider assistant system is further configured to generate the transcription and continue updating the transcription as additional voice data from the one or more microphones is received, whilst obtaining at least one of: information and generated content resulting from responses to one or more prompts from the set of prompts.

Optionally, the one or more processors further comprise an orchestrator module, the orchestrator module configured to: select the prompts which form the set of prompts which are provided to the trained language model for processing; control the provision of the transcription and/or the voice data to the trained language model; process the responses to one or more prompts from the set of prompts; determine if one or more of the responses are for further processing as inputs to the trained language model in a subsequent inference of the trained language model and/or as inputs to a software module; control one or more subsequent inferences of the trained language model to cause further processing of the responses and/or controlling the software module to process the one or more responses.

Optionally, one or more of the first subset of prompts relate to asking the patient for consent to store the voice data and/or generate the transcription, wherein the system is configured to determine whether the patient provides their consent based on the voice data relating to the patient voice; wherein if the system determines that the patient does not provide their consent, the healthcare provider assistant system prohibits the generating of the transcription of the voice data, storage of the voice data and the providing of the transcription and/or providing the voice data to the trained language model.

Optionally, the healthcare provider system is implemented on an electronic device; wherein the one or more of the first subset of prompts relates to asking the patient for consent for the voice data and/or transcript to be processed non-locally; wherein the one or more processors are configured to determine whether the patient provides their consent for the voice data and/or the transcription to be processed non-locally; wherein if the patient provides their consent then the healthcare provider assistant system is permitted to use an external trained language model not stored on the one electronic device as the trained language model; wherein if the patient does not provide their consent then the healthcare provider assistant system is restricted to using a language model stored on the electronic device.

Optionally, the healthcare provider assistant system is further configured to:
receive a request to register the first voice as the healthcare provider voice; obtain a sample voice recording of the first voice; calculate voice embeddings to determine features of the first voice; store the voice embeddings as being associated with the first voice; use the voice embeddings to recognise the first voice as the healthcare provider voice in the voice data.

Optionally, the healthcare data comprises data for providing a prescription recommendation; wherein the trained language model is configured to access one or more data stores which store prescription information to provide the prescription recommendation.

Optionally, the healthcare data comprises data for providing a referral recommendation for the patient; wherein the trained language model is configured to access one or more data stores which store referral information to provide the referral recommendation.

Optionally, the healthcare data comprises treatment guidelines for the patient; wherein the trained language model is configured to access one or more data stores which store treatment information to provide the treatment guidelines.

Optionally, the healthcare data comprises medical sensor data from at least one medical sensor.

Optionally, the healthcare data comprises diagnosis data.

Optionally, the healthcare data comprises electronic medical records of the patient.

Optionally the healthcare provider assistant system is further configured to extract key information from the voice data and generate a summary comprising the key information, wherein the key information comprises one or more of: the patient's name;
the reason the patient consulted the healthcare provider; symptoms described by the patient; pre-existing conditions of the patient; medications which the patient already takes;
examinations conducted by the healthcare provider; diagnosis which the healthcare provider gives; prescriptions which the healthcare providers gives; referrals for further examination the healthcare provider gives.

Optionally, the healthcare provider assistant system is further configured to send the summary of the transcription to another healthcare provider assistant system and/or to a separate electronic device.

Optionally, the healthcare provider assistant system is further configured to generate a formatted document based on the voice data and/or the transcription, comprising one or more of: a diagnosis, a summary of session, a recommended treatment, a recommended prescription.

Optionally, the system additionally comprises one or more speakers, wherein the system is further configured to provide responses to one or more prompts from the set of prompts as text-to-audio output via the one or more speakers.

According to an aspect of the present invention there is provided an emergency vehicle comprising the healthcare provider assistant system of any preceding paragraph.

According to an aspect of the present invention there is provided a computer-implemented method for assisting a healthcare provider, the method comprising: receiving voice data from one or more microphones; recognising a first voice as a healthcare provider voice previously registered; assigning a second voice as a patient voice; generating a transcription of the voice data, wherein the transcription identifies words spoken by the healthcare provider voice and words spoken by the patient voice; providing the transcription and/or the voice data to a trained language model; providing a set of prompts to the trained language model; wherein the set of prompts comprise: a first subset of prompts associated with the healthcare provider voice; wherein each prompt comprises one or more tasks for the trained language model to complete, using the transcription and/or voice data; wherein at least one prompt of the first subset of prompts relates to obtaining healthcare data based on words spoken by the healthcare provider voice; processing the transcription and/or the voice data and the set of prompts using the trained language model to provide responses to one or more prompts from the set of prompts.

Optionally, the method further comprises: storing the transcription, voice data, response to one or more prompts from the set of prompts on a first memory of a first healthcare provider assistant system arranged to perform the method.

Optionally the method further comprises: transferring the transcription, voice data, responses from the first healthcare provider assistant system to a second healthcare provider assistant system arranged to perform the method.

Optionally, the first healthcare provider assistant system is provided in an emergency vehicle, and the second healthcare provider assistant system is provided in a room of a building.

The following numbered clauses provide further exemplary implementations.
1. A healthcare provider assistant system comprising:
   one or more processors collectively configured to:
   receive voice data from one or more microphones;
   recognise a first voice as a healthcare provider voice previously registered with the healthcare provider assistant system;
   assign a second voice as a patient voice;
   generate a transcription of the voice data, wherein the transcription identifies words spoken by the healthcare provider voice and words spoken by the patient voice;
   provide the transcription and/or the voice data to a trained language model;
   provide a set of prompts to the trained language model;
   wherein the set of prompts comprise:
      a first subset of prompts associated with the healthcare provider voice;
      wherein each prompt comprises one or more tasks for the trained language model to complete, using the transcription and/or voice data;
   wherein at least one prompt of the first subset of prompts relates to obtaining healthcare data based on words spoken by the healthcare provider voice;
   wherein the trained language model is arranged to process the transcription and/or the voice data and the set of prompts to provide responses to one or more prompts from the set of prompts.
2. A healthcare provider assistant system as described in clause 1, wherein the set of prompts additionally comprise a second subset of prompts associated with the patient voice.
3. A healthcare provider assistant system as described in clause 1, wherein at least one prompt of the second subset of prompts relates to obtaining healthcare data based on words spoken by the patient voice.
4. A healthcare provider assistant system as described in any preceding clause, additionally comprising a display, wherein the display is configured to display at least one of: information and generated content resulting from responses to one or more prompts from the set of prompts, and optionally wherein the healthcare provider assistant system is
5. A healthcare provider assistant system as described in clause 4, further configured to display the information relating to the responses to one or more prompts on the display whilst receiving additional voice data from the one or more microphones and continuing to generate the transcription of the voice data.
6. A healthcare provider assistant system as described in any preceding clause, further configured to generate the transcription and continue updating the transcription as additional voice data from the one or more microphones is received, whilst obtaining at least one of: information and generated content resulting from responses to one or more prompts from the set of prompts.
7. A healthcare provider assistant system as described in any preceding clause, wherein the one or more processors further comprise an orchestrator module, the orchestrator module configured to:
   select the prompts which form the set of prompts which are provided to the trained language model for processing;
   control the provision of the transcription and/or the voice data to the trained language model;
   process the responses to one or more prompts from the set of prompts;
   determine if one or more of the responses are for further processing as inputs to the trained language model in a subsequent inference of the trained language model and/or as inputs to a software module;
   control one or more subsequent inferences of the trained language model to cause further processing of the responses and/or controlling the software module to process the one or more responses.
8. A healthcare provider assistant system as described in any preceding clause, wherein one or more of the first subset of prompts relate to asking the patient for consent to store the voice data and/or generate the transcription, wherein the system is configured to determine whether the patient provides their consent based on the voice data relating to the patient voice;
   wherein if the system determines that the patient does not provide their consent, the healthcare provider assistant system prohibits the generating of the transcription of the voice data, storage of the voice data and the providing of the transcription and/or providing the voice data to the trained language model.
9. A healthcare provider assistant system as described in any preceding clause, wherein the healthcare provider system is implemented on an electronic device; wherein the one or more of the first subset of prompts relates to asking the patient for consent for the voice data and/or transcript to be processed non-locally; wherein the one or more processors are configured to determine whether the patient provides their consent for the voice data and/or the transcription to be processed non-locally;
   wherein if the patient provides their consent then the healthcare provider assistant system is permitted to use an external trained language model not stored on the one electronic device as the trained language model;
   wherein if the patient does not provide their consent then the healthcare provider assistant system is restricted to using a language model stored on the electronic device.
10. A healthcare provider assistant system as described in any preceding clause, further configured to:
   receive a request to register the first voice as the healthcare provider voice;
   obtain a sample voice recording of the first voice;
   calculate voice embeddings to determine features of the first voice;
   store the voice embeddings as being associated with the first voice;
   use the voice embeddings to recognise the first voice as the healthcare provider voice in the voice data.
11. A healthcare provider assistant system as described in any preceding clause, wherein the healthcare data comprises data for providing a prescription recommendation;
   wherein the trained language model is configured to access one or more data stores which store prescription information to provide the prescription recommendation.
12. A healthcare provider assistant system as described in any preceding clause, wherein the healthcare data comprises data for providing a referral recommendation for the patient;
   wherein the trained language model is configured to access one or more data stores which store referral information to provide the referral recommendation.
13. A healthcare provider assistant system as described in any preceding clause, wherein the healthcare data comprises treatment guidelines for the patient;
   wherein the trained language model is configured to access one or more data stores which store treatment information to provide the treatment guidelines.
14. A healthcare provider assistant system as described in any preceding clause, wherein the healthcare data comprises one or more of: medical sensor data from at least one medical sensor.
15. A healthcare provider assistant system as described in any preceding clause wherein the healthcare data comprises diagnosis data.
16. A healthcare provider assistant system as described in any preceding clause, wherein the healthcare data comprises electronic medical records of the patient.
17. A healthcare provider assistant system as described in any preceding clause, further configured to extract key information from the voice data and generate a summary comprising the key information, wherein the key information comprises one or more of:
   the patient's name;
   the reason the patient consulted the healthcare provider;
   symptoms described by the patient;
   pre-existing conditions of the patient;
   medications which the patient already takes;
   examinations conducted by the healthcare provider;
   diagnosis which the healthcare provider gives;
   prescriptions which the healthcare providers gives;
   referrals for further examination the healthcare provider gives.
18. A healthcare provider assistant system as described in clause 17, further configured to send the summary of the transcription to another healthcare provider assistant system and/or to a separate electronic device.
19. A healthcare provider assistant system as described in any preceding clause, further configured to generate a formatted document based on the voice data and/or the transcription, comprising one or more of: a diagnosis, a summary of session, a recommended treatment, a recommended prescription.
20. A healthcare provider assistant system as described in any preceding clause, wherein the system additionally comprises one or more speakers, wherein the system is further configured to provide responses to one or more prompts from the set of prompts as text-to-audio output via the one or more speakers.
21. An emergency vehicle comprising the healthcare provider assistant system of any preceding claim.
22. A computer-implemented method for assisting a healthcare provider, the method comprising:
   receiving voice data from one or more microphones;
   recognising a first voice as a healthcare provider voice previously registered;
   assigning a second voice as a patient voice;
   generating a transcription of the voice data, wherein the transcription identifies words spoken by the healthcare provider voice and words spoken by the patient voice;
   providing the transcription and/or the voice data to a trained language model;
   providing a set of prompts to the trained language model;
   wherein the set of prompts comprise:
      a first subset of prompts associated with the healthcare provider voice;
      wherein each prompt comprises one or more tasks for the trained language model to complete, using the transcription and/or voice data;
   wherein at least one prompt of the first subset of prompts relates to obtaining healthcare data based on words spoken by the healthcare provider voice;
   processing the transcription and/or the voice data and the set of prompts using the trained language model to provide responses to one or more prompts from the set of prompts.
23. The computer-implemented method of claim 22, further comprising:
   storing the transcription, voice data, response to one or more prompts from the set of prompts on a first memory of a first healthcare provider assistant system arranged to perform the method.
24. The computer-implemented method of claim 22, further comprising:
   transferring the transcription, voice data, responses from the first healthcare provider assistant system to a second healthcare provider assistant system arranged to perform the method.
25. The computer-implemented method of claim 24, wherein the first healthcare provider assistant system is provided in an emergency vehicle, and the second healthcare provider assistant system is provided in a room of a building.

### BRIEF DESCRIPTION OF THE DRAWINGS

Embodiments of the invention are further described hereinafter with reference to the accompanying drawings, in which:
Figure 1 shows a healthcare provider assistant system in accordance with an embodiment of the invention;
Figure 2A shows a healthcare provider assistant system in accordance with an embodiment of the invention;
Figure 2B shows part of a healthcare provider assistant system in accordance with an embodiment of the invention;
Figure 3 shows a room of a building in which a healthcare provider assistant system in accordance with an embodiment of the invention is provided;
Figure 4 shows a display of a healthcare provider assistant system in accordance with an embodiment of the invention;
Figure 5 shows a healthcare provider assistant system in accordance with an embodiment of the invention;
Figure 6 shows a healthcare provider assistant system in accordance with an embodiment of the invention;
Figure 7 shows a healthcare provider assistant system in accordance with an embodiment of the invention;
Figure 8 shows a healthcare provider assistant system in accordance with an embodiment of the invention;
Figure 9 shows a healthcare provider assistant system in accordance with an embodiment of the invention;
Figure 10A shows a first healthcare provider assistant system and a second healthcare provider assistant system in accordance with an embodiment of the invention;
Figure 10B shows a healthcare provider assistant system and an electronic device in accordance with an embodiment of the invention;
Figure 11 shows an emergency vehicle comprising a healthcare provider assistant system and a second healthcare provider assistant system in accordance with an embodiment of the invention;
Figure 12 shows a room of a building in which a healthcare provider assistant system in accordance with an embodiment of the invention;
Figure 13 shows a computer-implemented method in accordance with an embodiment of the invention;
Figure 14 shows part of a computer-implemented method in accordance with an embodiment of the invention;
Figure 15 shows part of a computer-implemented method in accordance with an embodiment of the invention; and
Figure 16 shows part of a computer-implemented method in accordance with an embodiment of the invention.

### DETAILED DESCRIPTION

Existing techniques for recording patient visits typically include the healthcare provider typing a summary into a personal computer or other electronic device and completing other actions such as referrals, prescriptions manually using their personal computer or electronic device. This may interrupt the flow of the patient's visit if the healthcare provider interrupts their interaction with the patient in order to type up the summary and/or perform other actions on their personal computer or electronic device.

Examples disclosed herein advantageously provide a healthcare provider assistant system which can transcribe the patient visit as well as performing actions such as determining a referral, a prescription and generating a formatted letter, which would otherwise need to be completed by the healthcare provider. In addition to this, the healthcare provider assistant system can assist with tasks during the patient's visit so that it collaborates with the healthcare provider in order to enhance the patient's visit.

According to examples disclosed herein, in order to integrate seamlessly with the healthcare provider's interaction with the patient, the healthcare provider assistant system is configured to automatically recognise the healthcare provider's voice and to recognise unknown voices as patient voices. By immediately recognising the healthcare provider's voice at the beginning of the session, this provides an improved interaction between humans and the system as a healthcare provider can provide minimal input in order to start the session with the healthcare provider assistant system. The system is arranged so that as soon as the session is started it can perform separate tasks, actions and analysis according to what each voice says.

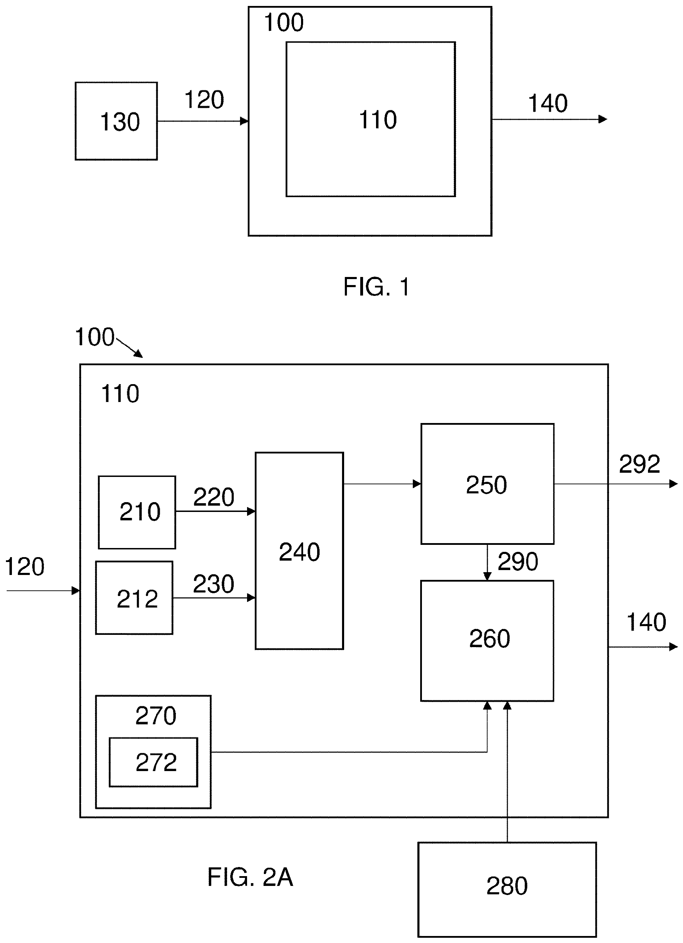

Figure 1 shows a healthcare provider assistant system 100 in accordance with an embodiment of the invention.

As shown in Figure 1, the healthcare provider assistant system 100 comprises one or more processors 110. The one or more processors 110 may be provided on a single electronic device or may be in a distributed network. Although not shown in Figure 1, the system 100 can also comprise memory, which can also either be stored on a single electronic device or can be distributed over a network. The memory can store computer program instructions (e.g. software) which is executed by the one or more processors 110. Alternatively the healthcare provider assistant system 100 does not comprise the memory. For example the system 100 may use external memory not associated with the system 100 in order to perform one or more functions.

As shown in Figure 1, the system 100 is configured to receive voice data 120 from one or more microphones 130. The system 100 may comprise an input means, such as an electrical input, to receive the voice data 120. Voice data 120 is received at the healthcare provider assistant system 100 in order to process the voice data 120 to generate a transcription of voice data 120. The voice data 120 may also be provided to a trained language model as described herein.

Figure 1 also shows providing, as an output of the healthcare provider assistant system 100, responses 140 to one or more prompts from a set of prompts. The set of prompts comprise prompts which comprise one or more tasks for a trained language model to complete. The trained language model uses the voice data 120 and/or the transcription to provide the responses 140 to the one or more prompts from the set of prompts.

The responses 140 may be provided at an output means of the healthcare provider assistant system 100. For example the output means can comprise an electrical output of the healthcare provider assistant system 100. The responses 140 to the one or more prompts from the set of prompts can be provided to a user, for example the healthcare provider, using a display, or other means. The responses 140 can also be used as further inputs to the trained language model in other inferences of the trained language model, or can be provided to other agents (for example other trained language models or other machine learning models) to generate further responses for other prompts. Inferences are to be understood as prediction or output generation cycles of machine learning models, and may also be referred to as calls of machine learning models. For example the responses 140 to the one or more prompts from the set of prompts can be used as further prompts for another inference of the trained language model or for another agent.

Figure 2A shows a healthcare provider assistant system 100 in accordance with an embodiment of the invention. In particular, Figure 2A shows operations or processes performed by the one or more processors 110 of the healthcare provider assistant system 100, as disclosed herein for example in Figure 1.

As shown in Figure 2A, the healthcare provider assistant system 100 receives voice data 120 as described herein. In a first operational block 210, the one or more processors 110 are arranged to recognise 210 a first voice 220. The first voice 220 is a voice of a healthcare provider previously registered with the healthcare provider assistant system 110. For example the one or more processors 110 are configured to use voice embeddings to recognise the first voice 220 as the healthcare provider voice in the voice data 120.

In a second operational block 212, the one or more processors 110 are arranged to assign 212 a second voice 230. The second voice 230 is assigned 212 as a patient voice. For example the one or more processors 110 may determine that no voice embeddings associated with or known to the healthcare provider assistant system 100 match the second voice 230 and therefore assigns the second voice as a patient voice. In other examples, the patient voice can be assigned and referred to as an unknown voice.

According to examples, the first operational block 210 and the second operational block 212 may be part of a single operational block. For example, the recognising 210 of a first voice 220 and the assigning 212 of a second voice 230 may be part of the same operation or process.

As shown in Figure 2A, the first voice 220 and the second voice 230 are provided to the third operational block 240, which is arranged to generate 240 a transcription 250 of the voice data 120. Using the information determined by the one or more processors 110 regarding which is the first voice 220 and the second voice 230, the transcription 250 identifies words spoken by the healthcare provider voice and the words spoken by the patient voice.

According to examples, the generation 240 of the transcription 250 is generated using an appropriate speech recognition model which takes audio data, such as the voice data 220, as input and outputs the detected spoken text. The speech recognition model is arranged to detect the number of speakers and identify which speakers are part of the transcript, and matches parts of the transcript spoken by different voices.

The speech recognition model can for example comprise a neural network architecture and according to some examples can comprise a transformer neural network. The voice data 220 can be fed into the neural network architecture as an input and the transcribed text is provided at the output of the neural network, and this can be done in end-to-end processing, which means that the raw audio waveform of the voice data 120 can be fed into the speech recognition model, without the requirement for feature engineering in which the raw audio would need to be converted into a data format suitable for inputting into the model.

According to examples, in addition to the speech recognition model, the generation of the transcription 250 can be completed in combination with a speaker diarization pipeline. The speaker diarization pipeline is configured to extract features of the voices detected by computing voice embeddings from segments of the voice data 120. The embeddings are high dimensional vectors that capture the unique characteristics of a particular voice. The embeddings can then be grouped into clusters based on similarity with each cluster assumed to represent a different voice. Clustering techniques such as K-means, agglomerative hierarchical clustering, or variational bayes can be used.

According to some examples, to get the final version of the transcription 250, timestamps from the speech recognition model are aligned with the timestamps from the diarization pipeline. In some situations, the timestamps from the speech recognition model do not match perfectly with the ones from the diarization pipeline, and so the one or more processors 110 can be configured to run an algorithm that finds a minimal distance in the time dimension between the timestamps from the speech recognition model and the timestamps from the diarization pipeline, thereby finding the closest alignment between them.

Following generation 240 of the transcription 250, the transcription 250 is provided 290 as an input to a trained language model 260. The trained language model 260 can comprise a large language model (LLM), and can be referred to as a large language model-based agent, or can simply be referred to as an agent.

As shown in Figure 2A, the transcription 250 may also be provided as a transcription output 292, which for example can be displayed on a display device, or sent elsewhere for storage and/or displaying on a display, or as text-to-speech playback.

The trained language model 260 is configured to receive input from one or more data sources. For example the transcription 250 can be one of the data sources. Another of the data sources provided to the trained language model 260 can be via a fourth operational block 270. According to examples disclosed herein the fourth operational block provides 270 a set of prompts 272 to the trained language model 260. The set of prompts 272 comprises a first subset of prompts associated with the healthcare provider voice. Each prompt comprises one or more tasks for the trained language model 260 to complete.

Therefore the trained language model 260 is configured to take at least the transcription 250 and at least one prompt from the set of prompts 272 that comprises the tasks for the language model 260 to complete and provides responses 140 to the one or more prompts from the set prompts.

According to examples, the one or more processors 110 are configured to provide the voice data 120 as an input to the trained language model 260. This can be done in addition to or instead of inputting the transcription 250 into the trained language model 260.

According to examples, the trained language model 260 can be configured to generate the transcription of the voice data 120.

For instance, the one or more prompts which are input into the trained language model 260 for obtaining the responses 140 are input at the same time as the voice data 120 which is to be transcribed using the trained language model 260.

According to examples, the trained language model 260 can comprise a multimodal model, which may process both audio data, for example the voice data 120, as well as text input, for example the set of prompts 272 and the transcription 250. The voice data 120, in other words the audio data, can be processed independently from the set of prompts 272 and the transcription 250, in other words the text data.

According to examples, a prompt that indicates a task to extract the transcription might be given as input to the model along with an audio snippet that needs to be transcribed. The trained language model 260 will then output the transcript of the audio snippet it received as input along with a specification about which voice or speaker said what at which timestamp.

The multimodal model may further be able to process image or video input or combinations of text, audio, image and video data. For example the multimodal model may receive images or video input relating to the consultation with the patient in addition to text and audio.

As shown in Figure 2A, according to examples the trained language model 260 can interact with healthcare data 280. According to examples, healthcare data 280 can be provided as another one of the data sources for the input of the trained language model 260. According to examples, the healthcare data 280 can be accessed by the trained language model 260 in order for the trained language model 260 to complete one or more of the tasks set by the one or more prompts. In Figure 2A, healthcare data 280 is shown as being separate to the healthcare provider assistant system 100. In other examples the healthcare data 280 may form part of the healthcare provider assistant system 100, for example it may be stored on the memory of the system 100.

Therefore, according to an aspect of the present invention, Figures 1 and 2A can be understood to show a healthcare provider assistant system 100 which comprises one or more processors 110 collectively configured to: receive voice data 120 from one or more microphones 130; recognise 210 a first voice 220 as a healthcare provider voice 330 previously registered with the healthcare provider assistant system; assign a second voice 230 as a patient voice.

The one or more processors 110 are further configured to generate a transcription 250 of the voice data. The transcription 250 identifies words spoken by the healthcare provider voice and words spoken by the patient voice and provides the transcription and/or the voice data to a trained language model 260. The one or more processors 110 are further configured to provide 270 a set of prompts 272 to the trained language model 260. The set of prompts 272 comprise a first subset of prompts associated with the healthcare provider voice. Each prompt comprises one or more tasks for the trained language model 260 to complete, using the transcription 250 and/or voice data 120. At least one prompt of the first subset of prompts relates to obtaining healthcare data 280 based on words spoken by the healthcare provider voice. The trained language model 260 is arranged to process the transcription 250 and/or the voice data 120 and the set of prompts 272 to provide responses 140 to one or more prompts from the set of prompts 272.

According to examples as described herein, the system 100 acting as an agent may implement a process where one or multiple trained language models 260, such as Large Language Models, are prompted to conduct different actions on different inputs. Therefore, for example, the system 100 according to embodiments of the invention may comprise multiple trained language models 260. The output from one trained language model 260 call might be part of the input of another trained language model 260 call. The agent controls and performs the entirety of the trained language model 260 calls stacked together to perform a set of actions. Therefore according to examples, the responses 140 to one or more prompts from the set of prompts may be provided as an input to another of the trained language models 260 of the multiple trained language models 260. Each of the multiple trained language model may also be provided with all of parts of the transcription 250, voice data 120, and healthcare data 280.

In examples of the system 100 comprising multiple trained language models 260, all of them may run on a local device or all on a cloud-server. In other examples, some of the trained language models 260 may run locally and some cloud-based (for example because different tasks require different computing power or have different data privacy restrictions)

According to examples, the trained language model 260 has been trained according to known training methods for language models, which includes using a text corpus of a wide variety of texts, which can be accessed for example via the internet. The text can be tokenised and then training can be performed. The architecture of the language model can comprise, for example, a transformer architecture, and the training can include the standard steps of processing a batch of text in a forward pass of the model, calculating the loss, backpropagating and then optimizing to minimise the loss. Techniques such as Masked Language Modelling (MLM) and Casual Language Modelling (CLM) may be used during this process.

The language model 260 may then be fine-tuned on more specific data for its purpose using supervised learning on input-output pairs.

Finetuning the language model 260 can comprise: improving the transcription through custom audio data: to accommodate for novel terms in medicine or local dialects / accents in spoken language, the model that performs the transcription might be fine-tuned. For that, a dataset of recorded voice data as well as the corresponding transcriptions is created. The language model will then be trained to generate the ground truth transcriptions based on the recorded voice data samples.

Fine-tuning the language model 260 can also comprise improving the formatting and quality of report drafts. This can be achieved by providing a dataset of clinic letters, prescriptions, referral letters etc. to fine-tune the model 260 to write those texts in appropriate format, detail and tone.

Fine-tuning the language model 260 can also comprise improving its capability to use specific text components to allow the language model orchestrator to parse its generated content. E.g. the language model could decorate its text with tag environments like <action> ... </action>, <action input> ... </action input>, <thinking> ... </thinking> etc. The training dataset would then also contain those tags that are also used in the right context. Those tags can be of any format for example any language, structure or genre.

According to examples disclosed herein, the healthcare provider assistant system 100 transcribes a conversation between a healthcare provider and the patient, and uses the transcription 250 and/or the voice data 120 to perform one or more tasks set according to the set of prompts 272, which involves obtaining healthcare data 280. The system 100 can therefore simultaneously provide a written record of the conversation, in other words the transcription 240, which is valuable for healthcare records and further healthcare of the patient, and perform tasks to assist the healthcare provider in providing healthcare to the patient.

By automatically recognising the voice of the healthcare provider and assigning an unknown voice as a patient, this results in a more efficient system which more quickly determines which voices are to be used to process tasks from the set of prompts. Recognising the healthcare provider voice and the unknown voice as the patient voice also means that the process of generating the transcription 250 and determining the responses 140 to the one or more prompts can be started more quickly. For example the system 100 may be configured so that the healthcare provider is able to press a single button or user interface icon associated with the system 100 to begin the generation of the transcription 250 and the providing of the responses 140. In other examples the system 100 can be configured to automatically recognise when a conversation between the healthcare provider and a patient has begun. For example the system 100, using the speech recognition model, may be arranged to automatically determine whether a voice command has been received from the healthcare provider to begin generation of the transcription 250 and the obtaining of the one or more responses 140.

According to examples, to enable speech recognition in parallel to recording of voice data 120, speech recognition can be conducted periodically with a sliding window (e.g. after each 5 seconds, the last 30 seconds will be transcribed). In situations of conflicting transcriptions 250 from a speech recognition inference or call, and a base transcription 250 that contains all the content transcribed so far, a merge may be conducted.

For example, the transcribed text of each subsequent voice data 120 snippet can be split into single sentences. For each of those sentences, the timestamp can be extracted that marks the beginning and the end of when this sentence was spoken. At the beginning and the end of each snippet there might be an incomplete sentence due to the cut of the voice data snippet.

According to examples, a base transcription 250 is provided that contains the transcription 250 of the whole patient consultation up to a particular time, i.e. since the system 100 was instructed to begin generating the transcription 250. The initial content of the base transcription 250 is the recognized completed sentences from processed voice data 120 snippets.

To merge a transcribed voice data snippet into the base transcription 250, for each of its completed sentences the timestamps are mapped with the timestamps of the sentences in the base transcription 250. If there is an approximate matching of the timestamps for a particular sentence, for example the difference between the timestamps of the start and end for both transcription versions is below a certain threshold, the sentence from the base transcription 250 will be replaced by the sentence extracted from the voice data snippet. If the last sentence from the base transcription 250 is not a complete sentence (i.e. the speech recognition model did not end with a punctuation mark), it can get replaced by content from the more recent voice data snippet.

Words or tokens from the newer voice data snippet that have a later timestamp than the last timestamp from the base transcription 250 are merged into the base transcription 250.

The current version of the base transcription 250 can be stored on a storage device or a cloud storage, such as the memory of the system 100. According to examples, the transcription 250 can be displayed on a display device and is updated as the conversation continues.

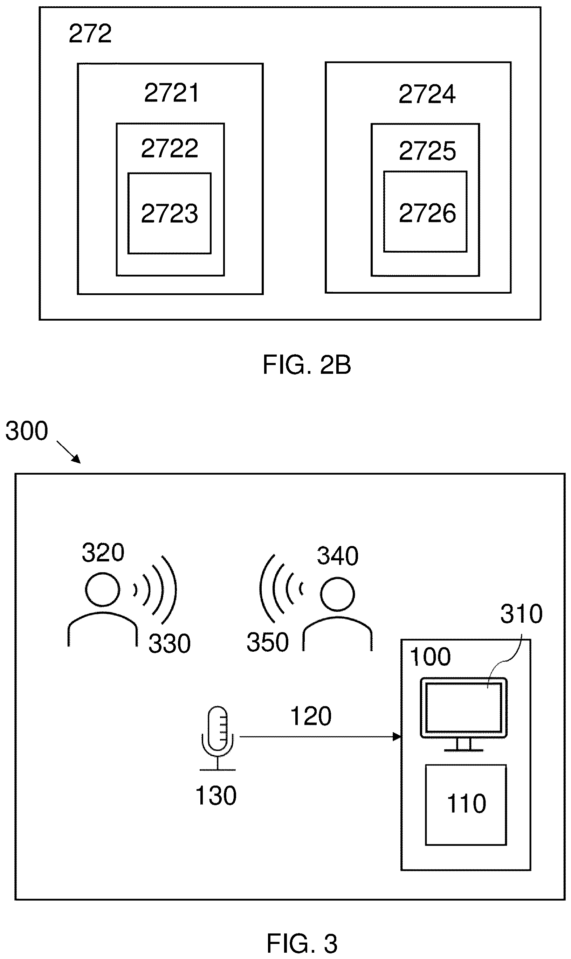

Figure 2B shows part of a healthcare provider assistant system 100 in accordance with an embodiment of the invention. In particular, Figure 2B shows an example set of prompts 272 which can be stored in a memory, for example they may be stored in a memory of the system 100.

As shown in Figure 2B, the set of prompts 272 comprises a first subset 2721 of prompts associated with the healthcare provider voice. The first subset 2721 of prompts comprises prompts 2722 which comprise one or more tasks 2723 for the trained language model 260 to complete, using the transcription 250 and/or the voice data 120.

It is to be understood that a prompt is a specific input given to a language model to generate a response. Each prompt comprises one or more tasks that together make up the entire prompt.

The set of prompts 272 shown in Figure 2B also comprise a second subset 2724 of prompts. The second subset 2724 of prompts are associated with the patient voice. The second subset 2724 are for example associated with the second voice 230 which the system 100 recognises as the patient voice.

The second subset 2724 of prompts also comprises prompts 2725 which comprise one or more tasks 2726. The one or more prompts 2725 and the tasks 2726 of the second subset 2724 may comprise at least one task which is different from the one or more prompts 2722 and tasks 2723 of the first subset 2721. The one or more prompts 2725 and tasks 2726 of the second subset 2724 may comprise at least one prompt 2725 and task 2726 which is the same as one or more prompts 2722 and tasks 2723 of the first subset 2721.

According to examples, at least one prompt 2725 of the second subset 2724 of prompts relates to obtaining healthcare data 280 based on words spoken by the patient voice.

It can therefore be understood that the first subset 2721 and the second subset 2724 comprise prompts 2722, 2725 which use the words spoken by the first voice 220 and the second voice 230 to provide the responses 140 to one or more prompts from the set of prompts 272.

In examples where at least some of the prompts 2722 from the first subset 2721 are different from the prompts 2725 of the second subset 2724, the prompts which are different between the two subsets 2721, 2724 may define actions which are specific to each of the healthcare provider voice and the patient voice.

For example a prompt 2722 of the first subset 2721, relating to the healthcare provider voice, may relate to actions that the healthcare provider wishes to make and have the system 100 assist them with in their role. For example the prompt may be a direct question to the healthcare provider assistant system 100, such as "find a drug which is suitable to prescribe for condition X" where "condition X" is a disease or illness. Alternatively, the healthcare provider assistant system 100 may automatically determine from the words spoken by the healthcare provider that it should find a drug to prescribe without directly being asked, and this may be defined by one of the prompts 2722 of the first subset 2721. For example the healthcare provider may say "I believe you have condition X", which is directed at the patient in their conversation, and the healthcare provider assistant system 100 may recognise that it has diagnosed the patient and that based on the specific words spoken and the condition X identified that it should find a drug to prescribe for condition X.

In another example, one or more prompts 2725 of the second subset 2724 may relate to specifically obtaining information from the patient voice. For example one or more prompts 2725 of the second subset 2724 may relate to finding patient health records based on the name given by the patient voice and other information such as date of birth. In another example, one or more prompts 2725 of the second subset 2724 may relate to providing a list of possible illnesses or conditions based on symptoms described by the patient voice. This prompt may be active over several inferences or calls of the trained language model or multiple trained language models, so that is it is updated as the patient continues to further describe their symptoms.

Advantageously the system 100 can therefore perform different actions based on what the healthcare provider and the patient says without the need for processing all of the words spoken by both the healthcare provider and the patient voice for each prompt across an entire set of prompts 272.

In some examples, one or more prompts 2722 and one or more prompts 2725 from each of subsets 2721, 2724 may be shared prompts or collaborative prompts. This means that the text spoken by both the healthcare provider voice and the patient voice may be used to provide responses 140 to the prompts in question.

For example where one or more of the prompts 2722, 2725 from both subsets 2721, 2724 relate to generating a formatted letter, the trained language model 260 may use information provided by both the healthcare provider voice and the patient voice to complete the letter. For example the patient voice may provide their name, age, address and other information such as symptoms and these may be formatted into the letter. The healthcare provider may provide information such as diagnosis, prescriptions, referrals and these may also be formatted into the latter.

Advantageously therefore the words spoken by the healthcare provider voice and the patient voice may be used collaboratively to provide a response to the one or more prompts, by categorising words being spoken by the healthcare provider and the patient voice. This provides more efficient processing of the generation of the responses, in this example a letter, as it can use specific words spoken from patient to complete part of the response and other parts spoken by the healthcare provider to complete other parts of the response.

Figure 3 shows a room 300 of a building in which a healthcare provider assistant system 100 in accordance with an embodiment of the invention is provided.

The building can example be a hospital, a general practitioner's surgery, an operating theatre, or other healthcare related setting.

As shown in Figure 3, a patient 320 and a healthcare provider 340 are shown. It is to be understood that other people may be present in the room 300, and that in some examples the system 100 is configured to only recognise, assign and transcribe the words spoken by the patient 320 and the healthcare provider 340. In some examples the system 100 is configured to recognise multiple healthcare provider voices previously registered with the healthcare provider system 100 and use each of these collectively as the first voice 220 for the purposes of generating the responses 140 to the first subset 2721 of prompts. According to examples the system 100 is configured to recognise multiple unknown voices and use each of these collectively as the second voice 230. For example the patient may be with a companion, carer or other person, and the system 100 is arranged to use the multiple unknown voices collectively as the second voice 230 for the purposes of providing responses 140 to one or more prompts from the second subset 2724 of prompts.

According to examples, if multiple voices are used collectively for the first voice 220 and or multiple voices are used collectively for the second voice 230, the system 100 is arranged to generate the transcription 250 which identifies all of the different voices as individuals. For example if two healthcare providers are used collectively as the first voice 220, the transcription 250 identifies them as different speakers and labels for example as "healthcare provider one" and "healthcare provided two" or gives their names. The same can apply if multiple voices are used collectively for the second voice 230.

According to examples "patient" is to be understood to be any person receiving healthcare from the healthcare provider 340, or alternatively may be a person speaking on behalf of the actual person who is being treated the healthcare. The patient voice according to examples disclosed herein may therefore comprise a voice spoken by a person representing the patient.

It is to be understood that the healthcare provider 340 is any professional or volunteer who provides healthcare. For example this can include any clinician, doctor, surgeon, nurse, paramedic, physiotherapist, psychiatrist, amongst others. As mentioned this can also include volunteers, such as volunteer paramedics.

Figure 3 shows waves representing the healthcare provider voice 350 and the patient voice 330. The patient voice 330 is the second voice 230 assigned by the healthcare provider assistant system 100. The healthcare provider voice 350 is the first voice 220 recognised by the healthcare provider assistant system 100.

As shown in Figure 3, one or more microphones 130 are provided in the room 300. The one or more microphones 130 receive the healthcare provider voice 350 and the patient voice 330 and provides voice data 120 containing data that represents the healthcare provider voice 350 and patient voice 330 in signal form. As shown in Figure 3, the voice data 120 is provided to the healthcare provider assistant system 100.

The healthcare provider assistant system 100 shown in Figure 3 is according to examples and embodiments described herein. In the example of Figure 3, the healthcare provider assistant system 100 comprises a display 310.

Although shown in a single room 300, it is to be understood that according to examples disclosed herein, the healthcare provider assistant system 100 can be distributed across different rooms and at least part of it may be accessed via cloud networking. The one or more microphones 130 may be in different rooms, including multiple different rooms, and can be in different rooms to the healthcare provider assistant system 100. According to some examples, the healthcare provider assistant system 100 comprises the one or more microphones 130.

The processing of the recorded audio of the interviews/consultations with patients as well as the processing of the transcription 250 and all additional data sources can happen on a device that is a located in the healthcare provider's room 300, on a central server within the hospital or other healthcare centre, on a cloud-server or with an arbitrary combination of all options mentioned previously. For example, the transcription 250 as well as the voice recognition could happen locally on a device within the healthcare provider's room 300 and the trained language model 260 may be located within a cloud-server. Parts of or all of the transcription 250 may be sent to the cloud-server for further processing after the transcription 250 was generated locally.

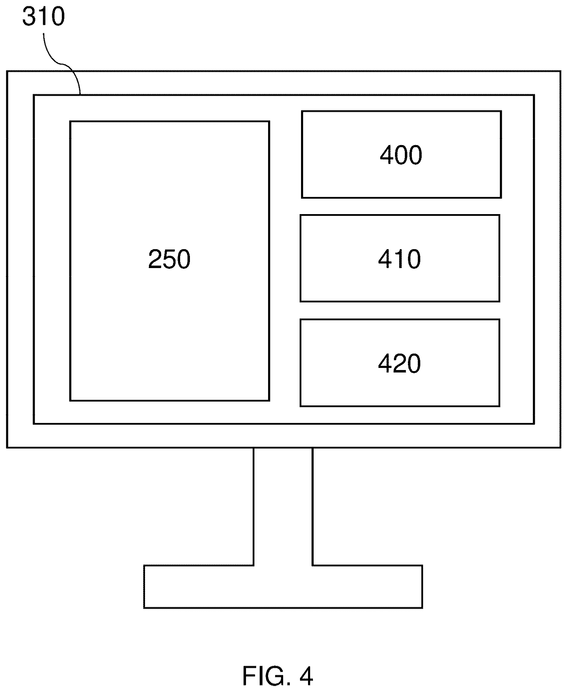

Figure 4 shows a display 310 of a healthcare provider assistant system 100 in accordance with an embodiment of the invention.

According to examples, the display 310 may be that shown in Figure 3, where it is provided in a room 300 with the healthcare provider 340 and the patient 320.

The display 310 according to examples disclosed herein can be part of a personal computer or other electronic device, and the healthcare provider assistant system 100 can comprise the personal computer or electronic device. The display 310 and the processors 110 of the system 100 or alternatively the processors of the electronic device or personal computer may be configured to receive input from users such as healthcare provider 340 via a touchscreen incorporated with the display 310, and/or a mouse and keyboard or other input device.

Figure 4 shows the display 310 displaying the transcription 250. The transcription 250 displayed on the display 310 can for example be the full transcription 250 generated by the system 100, or can be a part of the transcription 250 or a summarised version of the transcription 250.

In Figure 4, the display 310 is also displaying information 400. The information 400 can be any information resulting from the responses 140 to one or more prompts from the set of prompts 272. For example this can be a text response comprising information obtained by the trained language model 260. In some examples, the responses to the one or more prompts are provided as further inputs to other agents/machine learning models/language models. Therefore it is to be understood that the information resulting from the responses 140 includes information which has been provided dependent on the generation of the responses to the one or more prompts but the responses 140 themselves may have first been provided as input to one or more other agents and/or language models.

As shown in Figure 4, the display 310 is also displaying generated content 410. The generated content 410 can be any generated content resulting from the responses 140 to one or more prompts from the set of prompts 272. For example the generated content can comprise a letter generated by the trained language model 260. In some examples, the responses to the one or more prompts are provided as further inputs to other agents/machine learning models/language models. Therefore it is to be understood that the generated content resulting from the responses 140 includes content which has been generated dependent on the generation of the responses to the one or more prompts but the responses 140 may have been provided as input to another agent.

According to examples, the information 400 and generated content 410 may be provided as content comprising merged information. For example, a letter generated, which is an example of generated content 410, may contain information 400 obtained by the trained language model 260.

As shown in Figure 4, in this example the transcription 250 is displayed at the same time as the information 400 and the generated content 410. According to examples, the transcription 250 may be updated periodically or continuously/simultaneously with the display of the information 400 and generated content 410 resulting from the responses 140 to the one or more prompts from the set of prompts 272.

According to examples, the healthcare provider assistant system 100 is further configured to display at least one of the information 400 and the generated content 410 resulting from the responses 140 to one or more prompts on the display 310 whilst receiving additional voice data 120 from the one or more microphones and continuing to generate the transcription 250 of the voice data 120.

Therefore advantageously the system 100 is arranged to provide the transcription in parallel to providing at least one of information 400 and generated content 410 resulting from the responses 140 to the one or more prompts, providing an improved user interface which provides information to the healthcare provider whilst the consultation with the patient 320 is ongoing and whilst the transcription 250 continues to be generated.

According to examples, the system 100 may be configured to highlight the at least one of the information 400 and the generated content 410 in one or more ways. For example the system 100 may be configured to issue an audible chime via a speaker associated with the system 100 to indicate that at least one of the information 400 and the generated content 410 has been displayed on the display 310. For example system 100 may be configured to highlight at least one of the information 400 and the generated content 410 on the display 310 in a way which distinguishes it from the transcription 250 and other information or content displayed on the display 310. The highlighting may include for example displaying at least one of the information 400 and the generated content 410 at a different size, colour, or boldness compared to the transcription 250 and other information or content displayed on the display 310.

Highlighting at least one of the information 400 and the generated content 410 resulting from the responses 140 may include dimming the rest of the display 310 or the content displayed on the display apart from the information 400 and/or the generated content 410 resulting from the responses 140.

According to some examples, in order to do not distract the healthcare provider 340 and to highlight the information 400, the healthcare provider assistant system 100 may be configured to inhibit updating of the transcription 250 on the display 310 whilst at least one of the information 400 and the generated content 410 resulting from the responses 140 is displayed on the display 310. Although the updating of the transcription 250 is inhibited on the display 310, the system 100 may be configured to continue generating the transcription 250. For example the system 100 may continue to generate the transcription 250 in the background but inhibit displaying the updated transcription 250. This is advantageous in that it both saves processing power but also highlights the information 400 and/or generated content 410 to the user without the distraction of the transcription 250 continuing to update in the background. According to examples the inhibiting of the updating of the transcription 250 may be for a prescribed time limit before the system 100 resumes updating the transcription of the display 310.

According to examples disclosed herein, the transcription 250 may be updated and continuously generated whilst the trained language model 260 provides assistive functionality to the healthcare provider 340. The healthcare provider assistant system 100 may therefore be further configured to generate the transcription 250 and continue updating the transcription 250 as additional voice data 120 from the one or more microphones is received, whilst obtaining at least one of: information 400 and generated content 410 resulting from responses to one or more prompts from the set of prompts.

Advantageously the healthcare provider assistant system 100 can therefore provide real-time assistance whilst also generating a transcription 250 of the interaction between the healthcare provider 340 and the patient 320. This is achieved by providing generation of the transcription 250 in parallel with the providing of information and/or generated content resulting from the responses to the one or more prompts from the set of prompts 272. This may be further improved by using quantised versions of the speech recognition model and/or the trained language model 260 and/or other agents involved as described herein.

As shown in Figure 4, the display 310 also displays at least one user interface element 420. The at least one user interface element 420 may comprise any element which the user of the system 100, such as healthcare provider 340, can interact with and/or which can display information for the user. For example, the at least one user interface element 420 may comprise an element which the user can select to start or stop recording of the voice data 120 and the generation of the transcription 250. The at least one user interface element may comprise information about the patient obtained prior to the recording of the voice data 120 and generating of the transcription 250. For example the information can include any one or more of: the patient's name, age, gender, previous visits, planned future visits, medical history including any medications taken.

The at least one user interface element 420 can also comprise a text input element, configured to enable the user to enter text, for example to take personal notes in addition to the generating and recording of the transcription 250.

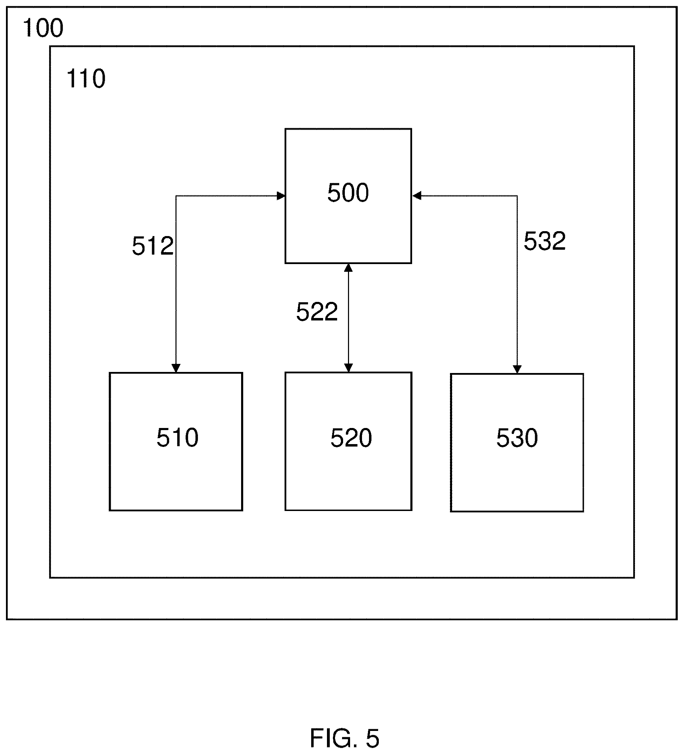

Figure 5 shows a healthcare provider assistant system 100 in accordance with an embodiment of the invention.

As shown in Figure 5, the healthcare provider assistant system 100 comprises one or more processors 110 as described herein according to the various examples. The one or more processors 110 are configured as in the other examples disclosed herein.

Figure 5 shows the one or more processors 110 comprising software blocks/modules 500, 510, 520, 530. One or more of the software blocks/modules can be carried out in a single processor or may be distributed across different processors of the one or more processors 110.

Software block 500 comprises an orchestrator module, 500. The orchestrator module 500 is configured to coordinate and control various multiple services or processes to achieve a particular task. It is configured to handle the sequencing, error handling, and integration of other various software blocks/modules 510, 520, 530. In some examples the orchestrator module 500 may be referred to as a controller.

Software block 510 comprises a first inference of the trained language model 260. This can also be referred to as a first call of the trained language model 260. The orchestrator module 500 is configured to execute the trained language model 260 first inference 510 using the set of prompts 272 and parses and processes the output from it. The orchestrator model 500 is arranged to provide inputs to the first inference 510 and receive outputs from the first inference 510 via first inference interface 512. The orchestrator module 500 may also include computer program instructions which are configured to select the prompts which form the set of prompts 272 which are provided to the trained language model 260 for processing. Depending on the responses 140 to the one or more prompts provided as the output from the first inference 510, the orchestrator module 500 is configured through computer program instructions to determine if one or more of the responses 140 are for further processing as inputs to the trained language model 260 in a subsequent inference. For example, the orchestrator module 500 may be configured to determine that the first inference 510 has created an intermediate summary of information from the transcription 250 and is configured to use a subsequent inference, such as a second inference 520 of the trained language model 260 creates a specific format of the information, such as a formatted letter. To do this the orchestrator module 500 may be configured to select particular prompts as the set of prompts 272 provided to the second inference 520.

As mentioned software block 520 is a second inference of the trained language model 260. The second inference 520 is arranged to receive inputs and provide outputs to the orchestrator module 500 via second inference interface 522. For example the inputs to the second inference 520 may be prompts from a set of prompts 272 selected by the orchestrator module 500 which may comprise responses 140 from a previous inference of the trained language model 260, such as from first inference 510.

The second inference 520 inputs may also comprise at least part of the transcription 250 and/or part of the voice data 120. Second inference 520 is arranged to provide responses 140 which may include generated content and/or information obtained, and may provide these to the orchestrator module 500.

Software block 530 may comprise a software module which isn't the trained language model 260. For example the software module 530 may comprise computer program instructions to carry out one or more particular tasks or may be an application. Software module 530 is arranged to receive inputs and provide outputs to the orchestrator module 500 via software module interface 532.

In an example, software module 530 may comprise a text formatter application, which may take, for example, responses 140 from the second inference 520 and/or the first inference 510 and process the responses to produce, for example, a formatted letter. In other examples the software module 530 may comprise a database search, which may for example search for electronic medical records, an internet search, amongst other examples.

In other examples the software module 530 can comprise a trained language model which is not the trained language model 260, such as a Large Language Model.

The first inference 510, second inference 520 and software module 530 can be called in any order or simultaneously. Additionally the different inferences of the trained language model 260 and the software module 230 may be called without dependence upon each other.

Therefore it is to be understood that in one or more examples the one or more processors 110 further comprise the orchestrator module 500, the orchestrator module being configured to: select the prompts which form the set of prompts 272 which are provided to the trained language model 260 for processing; control the provision of the transcription and/or the voice data to the trained language model 260; process the responses 140 to one or more prompts from the set of prompts; determine if one or more of the responses 140 are for further processing as inputs to the trained language model 260 in a subsequent inference of the trained language model 260 and/or as inputs to a software module 230; control one or more subsequent inferences of the trained language model 260 to cause further processing of the responses 140 and/or controlling the software module to process the one or more responses 140.

According to one or more examples the orchestrator module 500 is configured to manage or control any process and/or action performed by the control system 100. For example, the orchestrator module 500 may be further configured to control displaying of information 400 relating to the responses 140 to one or more prompts on the display 130 whilst receiving additional voice data 120 from the one or more microphones 130 and continuing to generate the transcription 250 of the voice data.

According to one or more examples, the orchestrator module 500 is configured to control generating of the transcription 250 and continue updating the transcription 250 as additional voice data 120 from the one or more microphones 130 is received, whilst obtaining at least one of: information 400 and generated content 410 resulting from responses 140 to one or more prompts from the set of prompts 272.

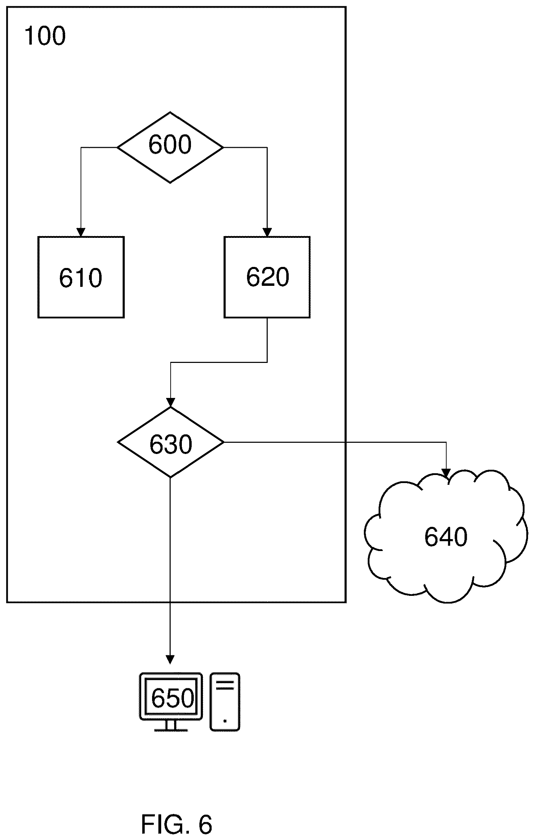

Figure 6 shows a healthcare provider assistant system 100 in accordance with an embodiment of the invention.

In particular, Figure 6 shows example operations or processes which the one or more processors 110 are configured to carry out.

Block 600 is a decision block where the one or more processors 110 are configured to determine an answer to a particular question based on the voice data 120 and/or the transcription 250. In particular, according to the example shown in Figure 6, decision block 600 relates to determining whether the patient 320 provides their consent to store the voice data 120 and/or generate the transcription 250. Decision block 600 may be triggered or activated when one or more of the first subset of prompts 2721 relates to asking the patient 320 for consent to store the voice data 120 and/or generate the transcription 250. For example, based on information determined from the transcription 250 and/or one or more inferences of the trained language model 260, it may be determined that the healthcare provider 340 has asked the patient 320 for their consent.

The system 100 is configured to then determine 600 in decision block 600 whether the patient 320 provides their consent based on the voice data 120 relating to the patient voice 330. For example the one or more processors 110 may use the trained language model 260 or other computer program instructions to determine whether the patient 320 provides their consent. The one or more processors 110 may determine that the patient 320 has given a positive answer which indicates that they do give their consent or alternatively may give a negative or an unclear statement which is used to indicate that they do not give their consent. In cases where the statement by the patient 320 is unclear, it is assumed that they have not given their consent.

According to examples, if the system 100 determines that the patient 320 does not provide their consent, the healthcare provider assistant system 100 prohibits 610 the generating of the transcription 250 of the voice data 120, storage of the voice data 120 and the providing of the transcription 250 and/or providing the voice data 120 to the trained language model 260.

According to examples, the prohibiting 610 may cause the healthcare provider system 100 to become dormant or enter an idle state. The system 100 may only become active again via a specific control input provided to the system 100. This may be referred to as the system 100 operating in an inhibited state. This therefore advantageously provides privacy control to the patient 320.

If the patient 320 provides their consent, the system 100 may proceed to operate in a normal state 620 of operation in which it can perform its necessary functions, which may include at least the generation of the transcription 250 and the processing of the transcription 250 and/or the voice data 120 with the set of prompts 272 using the trained language model 260 to provide responses 140 to the one or more prompts.

According to some examples, the system 100 may continue to operate in the normal state 620 of operation without further interruption. According to other examples, particularly when the healthcare provider assistant system 100 is implemented on an electronic device 650 the system 100 may determine that one of more of the first subset of prompts relates to asking the patient for consent for the voice data 120 and/or the transcription 250 to be processed non-locally, where non-locally refers to processing external to the electronic device which has the healthcare provider system implemented on it.

Similar to decision block 600, the system 100 may be arranged to have a decision block 630 for determining whether the patient provides consent for the voice data 120 and/or transcription 250 to be processed non-locally.

The system 100 is arranged to use the transcription 250 and/or the voice data 120 to determine if the patient 320 gives their consent. For example the one or more processors 110 may use the trained language model 260 or other computer program instructions to determine whether the patient 320 provides their consent.

If the patient 320 provides their consent then the healthcare provider assistant system 100 is permitted to use an external trained language model 640 not stored on the electronic device 650 as the trained language model 260.

If the patient 320 does not provide their consent then the healthcare provider assistant system 100 is restricted to using a language model 260 stored on the electronic device 650.

In examples where the healthcare provider assistant system 100 is permitted to use an external trained language model 640, this may be via cloud computing. Allowing the use of external trained language models 640 may lead to the activation of or more additional trained language models for use in combination with one or more trained language models 250 stored on the electronic device 650.

This advantageously allows the patient 320 to benefit from the use of the healthcare provider assistant system 100 even if they are not comfortable with having their voice data 120 and other data processed non-locally. The system 100 therefore provides an option for the processing of their data to be local or self-contained, or on-device, so that the patient 320 is reassured as to where their data is being stored and processed. This also advantageously provides the option for the patient 320 to take advantage of increased processing ability by the activation of additional agents non-locally or off-device which may provide more enhanced information and/or enhanced generated content and more efficient providing of the responses 140.

According to examples where the system 100 comprises a display 310, system 100 may be configured to display an indication on the display 310 as to whether the patient 320 has provided their consent to store the voice data 120 and/or generate the transcription 250 and/or provide their consent for the voice data 120 and/or transcription 250 to be processed non-locally. This can therefore provide a visible indication which reassures the healthcare provider 340 and the patient 320 that the patient's requests have been correctly understood by the system 100.

According to examples, system 100 may be configured to record one or more parts of the voice data 120 which contain the responses by the patient 320 as to whether they provide their consent to store the voice data 120 and/or generate transcription 250 and/or provide their consent for the voice data 120 and/or transcription 250 to be processed non-locally. The records of the one or more parts of the voice data 120 containing the responses by the patient 320 may then be stored either on the electronic device 650 comprising the system 100 or elsewhere.

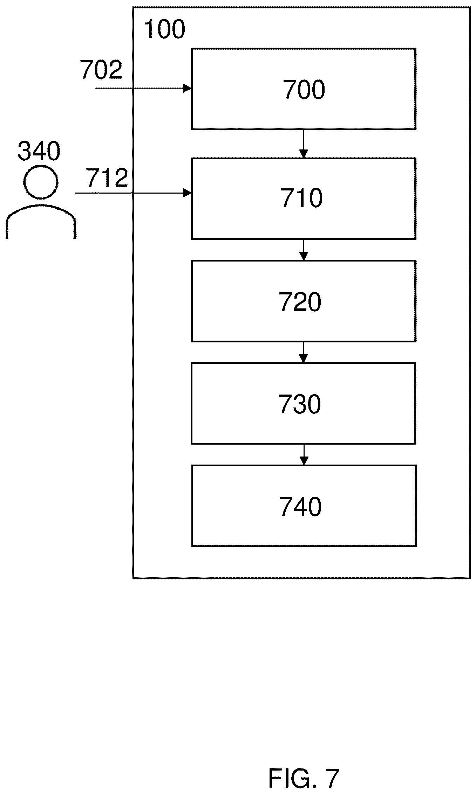

Figure 7 shows a healthcare provider assistant system 100 in accordance with an embodiment of the invention. In particular, Figure 7 shows example operations or processes which the one or more processors 110 are configured to carry out, and relates to registering voices as healthcare provider voices so that they subsequently use the healthcare provider assistant system 100 in the role of the healthcare provider voice 350.

In block 700, the one or more processors 110 receive 700 a request 702 to register the first voice as the healthcare provider voice. The request 702 may comprise an electrical signal input to the one or more processors 110 as shown in Figure 7. In other examples the request 702 may come from within the one or more processors 110. For the request 702 to be enacted, the system 100 may be configured to require that an administrative password or other security measure accompanies the request 702, to ensure that only authorised individuals can be registered as the healthcare provider voice 350.

Following the receiving 700 of the request 702, the one or more processors 110 are configured to obtain 710 a sample voice recording 712 of the first voice. In some examples the sample voice recording 712 may have been pre-recorded and provided to the system 100, and in other examples the system 100 may issue an instruction for a sample voice recording to be prepared by the individual registering the first voice as the healthcare provider voice. This instruction may be provided for example as a notification on a display 310 of the system 100, which may include a user interface for enabling recording of the sample voice recording 712.

Following the obtaining 710 of the sample voice recording 712, the one or more processors 110 calculate 720 voice embeddings to determine features of the first voice, and store 730 the voice embeddings as being associated with the first voice. At this point the system 100 is arranged so that it will recognise the first voice as a healthcare provider voice. The system 100 is configured to use 740 the voice embeddings to recognise the first voice as the healthcare provider voice in the voice data 120.

Therefore voices can be preregistered as healthcare provider voices for use with the system 100 and will automatically recognise unknown voices as patient voices. When using the system 100, a healthcare provider who is registered with the system 100 may identify themselves to the system for example by logging into the system 100 using a user interface. Logging in may be achieved by using a username and password, facial recognition, fingerprint recognition or other identification means. Alternatively the system 100 may be configured to automatically recognise when a registered healthcare provider voice is speaking and process the voice data 120 containing the healthcare provider voice.

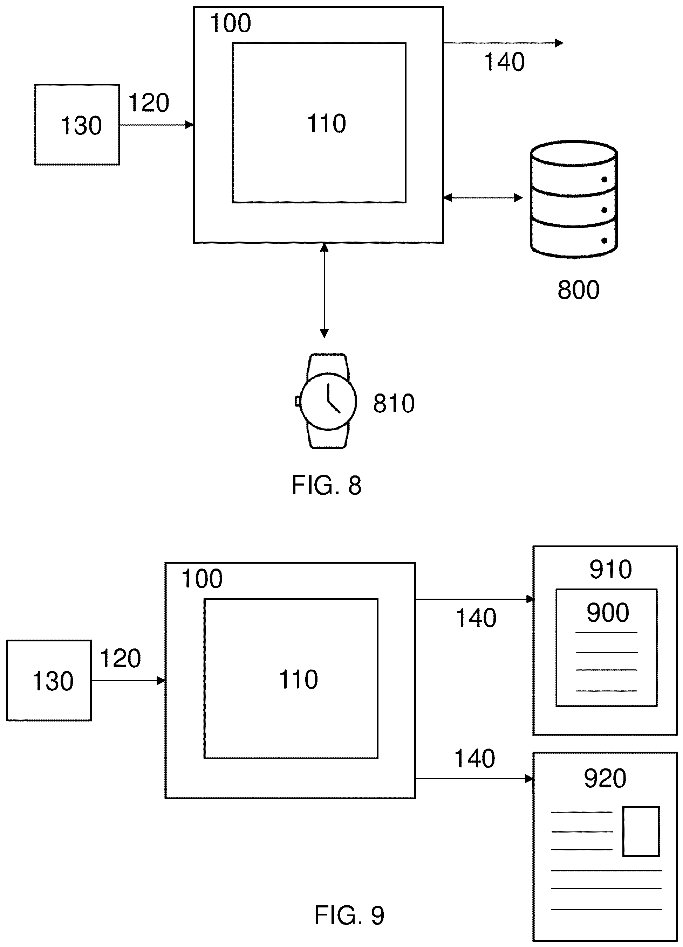

Figure 8 shows a healthcare provider assistant system 100 in accordance with an embodiment of the invention. The system 100 is as described according to examples disclosed herein comprising one or more processors 110 and configured to receive voice data 120 via one or more microphones 130 and provide responses 140 to one or more prompts from a set of prompts 272.

As shown in Figure 8, the healthcare provider assistant system 100 is configured to communicate and access with one or more data stores 800 which can provide healthcare data 280. Additionally or alternatively, healthcare provider assistant system 100 is configured to communicate with at least one medical sensor 810, which may include or more wearables. The at least one medical sensor 810 can provide medical data 280 to the system 100.

According to examples, the healthcare data 280 comprises data for providing a prescription recommendation and the one or more data stores 800 store prescription information. The trained language model is configured to access the one or more data stores 800 which store prescription information to provide the prescription recommendation.

For example, based on the transcription 250 and/or the voice data 120, the system 100 can detect when a healthcare provider 340 recommends that a particular type or category of medication is to be prescribed. Alternatively or in addition to this, the healthcare provider 340 may describe an illness or condition they believe the patient 320 has. Based on what the healthcare provider 340 said, the system 100, acting as an agent, can search for drugs matching the healthcare provider's recommendation and/or the illness/condition they have described. To do this, one or more prompts of the first subset 2721 include tasks 2723 to determine if the healthcare provider 340 is recommending a particular type or category of medicine and/or that they have described a particular illness/condition.

The search for the drugs can be conducted within a database, such as the one or more data stores 800, where data about available drugs are stored. The search can be enacted by the trained language model 260. The search may include a SQL database query, where for example the system 100 and/or trained language model 260 would generate an SQL query to interact with the data store 800. The search may include a vector database where an embedding-based similarity search between texts representing the drugs and a search query generated by the system 100 and/or trained language model 260 acting as an agent would be executed.

The search might result in a list of matching drugs that could be prescribed by the healthcare provider 340 based on what they said previously. This list of drugs might then be displayed on the display 310 of the system 100 where the healthcare provider 340 might then be able to select one of the displayed drugs or to confirm the selected drug in case only one search result exists.

If a specific drug exists in different dosages, the system 100 and/or trained language model 260 acting as an agent may use information from the medical history of the patient 320 to suggest the best-fitting dosage. For example if the patient 320 visited the healthcare provider 340 or another healthcare provider 340 already several times because of the same symptoms, the healthcare provider 340 may confirm the same diagnosis, and the patient 320 may mention during the consultation that they were satisfied with the medication previously prescribed. The system 100 and/or trained language model 260 acting as the agent may suggest the same dosage as for the previous visits of the patient 320.

If on the other hand the patient 320 has the same symptoms, and the healthcare provider 340 confirms the same diagnosis and drug as a treatment without mentioning the dosage and the patient 320 then mentions that they are not satisfied with the medication, then the system 100 and/or trained language model 260 acting as the agent might suggest another dosage as the initial search result.

According to examples, the display 310 may also incorporate an option for the healthcare provider 340 to refine the search in case the search by the system 100 and/or trained language model 260 did not lead to a satisfying result.

According to examples, if the healthcare provider selects or confirms at least one drug for prescription to the patient 320, the system 100 and/or trained language model 260 acting as the agent can automatically draft the prescription form.

The system 100 can therefore advantageously assist in prescribing drugs.

According to examples disclosed herein, the healthcare data 280 can comprise data for providing a referral recommendation for the patient 320. In particular the one or more data stores 800 may store referral information and the trained language model 260 can be configured to access the one or more data stores 800 which store referral information to provide the referral recommendation.

The system 100 and/or the trained language model 260 acting as an agent can perform actions based on the transcription whenever it detects that the healthcare provider 340 mentions that they will refer the patient 320 to another healthcare provider 340 for further examination, using one or more prompts 2722 provided in the first subset 2721, which may contain one or more tasks 2723 relating to obtaining a referral.

The one or more data stores 800 which store referral information may comprise a database of available doctors or other healthcare providers which the system 100 and/or the trained language model 260 acting as an agent can search for, which were mentioned by the healthcare provider 340. Alternative if the mentioned healthcare provider for referral is not available (or no name was mentioned by the healthcare provider 340) it can search for healthcare providers 340 matching the specifications that were mentioned by the healthcare provider 340. For example, the search may be for available radiologists if the healthcare provider 340 mentioned to consult a radiologist for further examination.

If the healthcare provider 340 confirms the referral, for example using a user interface provided on the display 310 of the system 100, the system 100 and/or the trained language model 260 acting as an agent might draft a referral letter.

The system 100 can therefore advantageously assist in making referrals.

According to examples, the one or more data stores 800 may store treatment guidelines and the healthcare data may comprise treatment guidelines for the patient 320. The trained language model 260 may be configured to access the one or more data stores 800 which store the treatment information to provide the treatment guidelines. It is to be understood that treatment guidelines comprise any instructions for treating a patient, for example this includes instructions for drug dosages, prescription recommendations, surgery instructions, physical therapy instructions, amongst others.

According to examples, the one or more data stores 800 may store diagnosis data and the healthcare data 280 may comprise a diagnosis for the patient 320. The trained language model 260 may be configured to access the one or more data stores 800 which store diagnosis data to provide the diagnosis.

According to examples, the one or more data stores 800 may store electronic medical records of the patient 320 and the healthcare data 280 may comprise the electronic medical records for the patient 320. The trained language model 260 may be configured to access the one or more data stores 800 to provide the electronic medical records.

According to examples as described herein wherein the healthcare data 280 comprises treatment guidelines and/or diagnosis data and/or electronic medical records, the system 100 and/or the trained language model 260 acting as agent can retrieve the information from the one or more data stores 800, for example in a Retrieval Augmented Generation process, to assist in providing information such as treatment guidelines and diagnosis.

For example, if the system 100 and/or the trained language model 260 acting as an agent detects that the healthcare provider 340 asks a question, the agent will execute a search query within the one or more connected data stores 800 and will generate an answer to the query of the healthcare provider 340 based on the information and/or documents retrieved. The detection of the question may be via an input element on a user interface accessible through frontend software displayed on the display 310 or by a signalling word from the healthcare provider 340 such as "assistant" after which the healthcare provider 340 speaks their question.

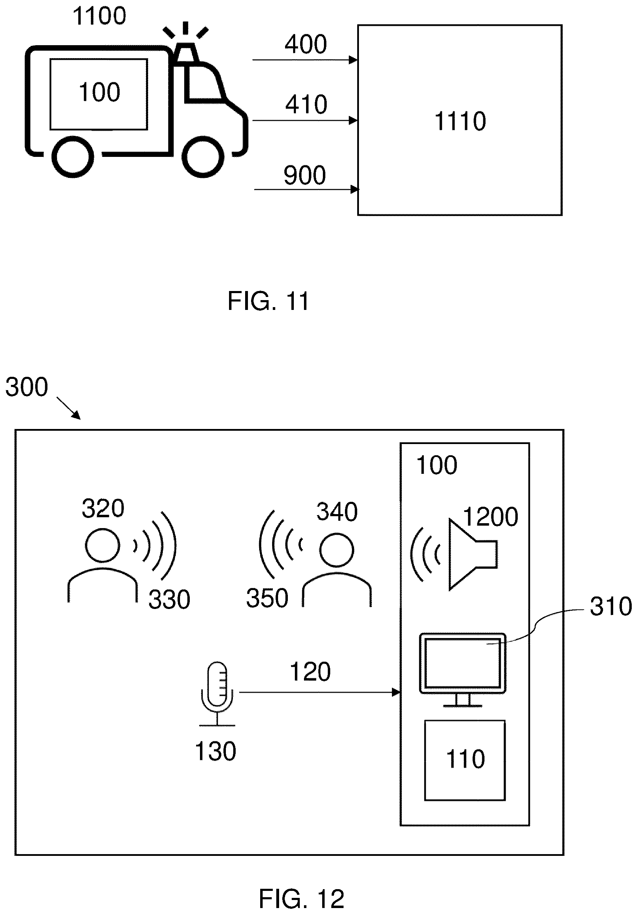

The answer to the healthcare provider's question may be provided in a written format on the display 310 or as an audio speech output using a Text-to-Speech model, such as described herein with reference to Figure 12.

According to examples, the system 100 and/or the trained language model 260 acting as an agent may periodically search in the one or more data stores 800 to retrieve information about treatment guidelines, or in other words next steps the healthcare provider 340 could conduct. For this, in a first step, the agent takes a summary of the patient's condition, the transcription 250 and previously conducted examinations, if any. The summary may be as described herein with reference to Figure 9. In a second step, the agent generates a search query to a vector store of the one or more data stores 800 that includes information taken from the summary mentioned above to find documents containing information about recommended follow-up actions. These follow-up actions, which may be referred to as treatment guidelines, can include potential diagnoses that match the medical condition/illness of the patient and the symptoms described by the patient, medications that are recommended given a specific diagnosis and considering specific drug intolerances or allergies extracted from the patient's medical health records, information about further examinations that are recommended given the patient's medical condition and the examination results previously recorded, and procedure instructions for treating the condition/illness.

According to examples disclosed herein, the healthcare data can comprise medical sensor data from at least one medical sensor 810. The at least one medical sensor 810 can be any sensor or group of sensors arranged to detect, measure, and monitor physiological signals or biological parameters from the patient 320. The sensors 810 are configured to convert any of physical, chemical, or biological stimuli into readable signals, which can then be used for diagnosis, monitoring, or treatment purposes in healthcare. Examples of sensors include: vital signs monitoring sensors which measure heart rate, blood pressure, body temperature, respiratory rate; Electrophysiological sensors, such as electrocardiogram (ECG) sensors, electroencephalogram (EEG) sensors; oxygen saturation sensors such as pulse oximeters that measure the oxygen level in the blood; glucose sensors, such as continuous glucose monitors (CGMs) that track blood sugar levels in diabetic patients; wearable sensors, such as those integrated into smartwatches, smart rings or fitness devices to monitor activity levels, sleep patterns, or detect irregular heart rhythms; implantable sensors, including devices placed inside the body to monitor chronic conditions, such as pacemakers or glucose sensors.

The system 100 according to examples disclosed herein may comprise an interface to interact with electronic medical records of the patient 320, and the at least one sensor 810, including wearables as shown in Figure 8, and/or a patient information system of a hospital or other healthcare centre. By using this interface, the system 100 according to examples disclosed herein can enhance a generated summary as described herein with reference to Figure 9, with information about the patient's medical history, his general health condition and/or results from previous examinations with other healthcare providers, including results using the at least one sensor 810 or other medical equipment.

Figure 9 shows a healthcare provider assistant system in accordance with an embodiment of the invention. As shown in Figure 9, the system 100 is as described according to examples disclosed herein comprising one or more processors 110 and configured to receive voice data 120 via one or more microphones 130 and provide responses 140 or more prompts from a set of prompts 272.

Figure 9 shows a summary 900 which has been generated by the system 100. According to examples, the summary 900 can be generated as one of the responses 140 to one or more prompts from the set of prompts 272. In other examples, the summary 900 is generated as a result of the one or more responses 140. For example one or more responses 140 to the prompts may be provided to a software module 530 in reference to Figure 5, configured to generate the summary.

The summary 900 may comprise key information extracted from the transcription 250 and/or the voice data 120 by the system 100. The key information can comprise, for example: the patient's name; the reason the patient consulted the healthcare provider; symptoms described by the patient; examinations conducted by the healthcare provider; diagnosis which the healthcare provider gives and/or the system 100 recommends; prescriptions which the healthcare providers gives and/or the system 100 recommends; referrals for further examination the healthcare provider gives and/or the system 100 recommends.

The summary 900 can provide information of what happened during the patient consultation. This can happen whilst the consultation is taking place between the healthcare provider 340 and the patient 320, and the summary 900 can be refined by the system 100 multiple times during the conversation in order to have an up-to-date summary of the conversation, which can be displayed on the display 310. This summary 900 can then be used to review what has been discussed during the patient consultation, and also to use it for an efficient handover of the patient from one healthcare provider 340 to another.

In Figure 9, the summary 900 is provided as a formatted document 910. The formatted document 910 may be generated as one of the responses 140 to a prompt from the set of prompts 272, or may be generated by a software module as a result of the responses 140 to the set of prompts 272.

The formatted document can comprise a draft for a patient letter, a referral letter or any other document that aims to provide information about the patient's visit to other entities.

The draft of the formatted document 910 can be displayed on the display 310 of the system 100 and there can be an input interface implemented for the healthcare provider 340 to modify the draft generated by the system 100. The input interface can be based on a frontend of the system 100 which the healthcare provider 340 can interact with via keyboard and mouse and/or touchscreen, and in other examples can be based on interaction by speaking to the system 100 via the one or more microphones 130. The system 100 can comprise functionality implemented to output the formatted document 910 in different formats such as PDF, DOCX, ODF.

As shown in Figure 9, the system 100 can generate other formatted documents 920 based on the voice data and/or the transcription 250. For example the formatted document 920 may be generated as one of the responses 140 to a prompt from the set of prompts, or may be generated by a software module as a result of one or more of the responses 140 to the prompts. The formatted document 920 can comprise one or more of: a diagnosis, a summary of session, a recommended treatment, a recommended prescription. As with the formatted document 910, the formatted document 920 may be provided as a draft for review by the healthcare provider 340.

An example of drafting a referral letter can comprise the following steps.

Firstly, the system 100 may detect from the transcription, and using the set of prompts, that the healthcare provider 340 wants to refer the patient 320 to another healthcare provider 340 and therefore triggers a referral letter drafting action.

Then the trained language model may be provided with a set of prompts 272 in an inference or call which includes one or more prompts to extract and summarise all relevant information for the referral from the transcription and potential further data sources, such as the voice data 120.

Then from the summary 900, the trained language model 260 can extract information about what kind of healthcare providers the patient 320 should be referred to. For example the trained language model 260 may use one or more data stores 800 as discussed herein with reference to Figure 8.

The trained language model 260 is prompted, for example using another set of prompts in another inference, to generate a request to a database or web resource stored on a data store 800 that contains available healthcare providers. The request should contain information about which kind of healthcare provider the patient 320 should be referred to.

The generated request can be executed by the orchestrator module 500 as described herein with reference to Figure 5, and a result that contains a list of best matching healthcare providers is returned.

The orchestrator module 500 takes the response of the request and displays it on the display 310 waiting for the healthcare provider 340 to select one of them.

After the healthcare provider 340 selects the healthcare provider from the displayed list of healthcare providers, the trained language model 260 is prompted, for example using another set of prompts 272, to transform the summarised information and the information about the selected healthcare provider into a referral letter formatted document 920.

The orchestrator module can take the generated referral letter draft and display it to the healthcare provider 340 on the display 310, where they can edit or approve it.

After the healthcare provider 340 approves the referral letter, it may be stored in a database, for example one or more data stores 800, where it is available for further actions such as printing, emailing the letter.

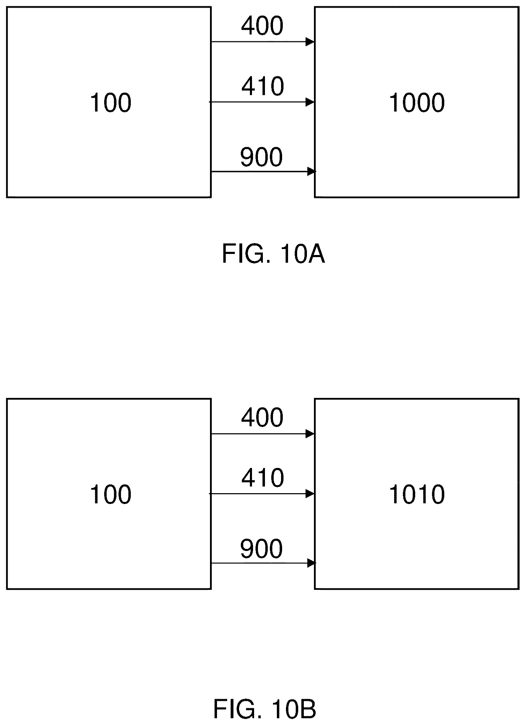

Figure 10A shows a first healthcare provider assistant system and a second healthcare provider assistant system in accordance with an embodiment of the invention.

Figure 10B shows a healthcare provider assistant system and an electronic device in accordance with an embodiment of the invention;

Systems 100 according to examples disclosed herein can comprise a communication interface to communicate with other systems, including other healthcare provider assistant systems 100, and other systems such as hospital information systems, clinical information systems. For example, generated content and/or information obtained from the system 100 such as prescription forms, clinic letters, extracted metadata or referral letters can be transferred to other systems through this interface. The interface may be for example, a unidirectional interface (such as a REST API) or a bidirectional / event-based interface (such as Webhooks, Websockets or Server-Sent Events) that allow information to be pushed to other systems.

As shown in Figure 10A and Figure 10B, the system 100 is further configured to send at least one of: information 400 and generated content 410 resulting from responses to one or more prompts from the set of prompts to another healthcare provider assistant system 1000 and/or to a separate electronic device 1010.

As shown in Figure 10A and Figure 10B, the system 100 is further configured to send the summary 900 as described herein with reference to Figure 9 to another healthcare provider assistant system 1000 and/or to a separate electronic device 1010.

In some examples, the healthcare provider assistant system 100 may be provided in an emergency vehicle. Figure 11 shows an emergency vehicle 1100 comprising a healthcare provider assistant system 100 in accordance with an embodiment of the invention. Figure 11 also shows a second healthcare provider assistant system 1110 in accordance with an embodiment of the invention. As described herein, the healthcare provider assistant system 100 of the emergency vehicle 1100 can send at least one of: information 400 and generated content 410 resulting from responses to one or more prompts from the set of prompts to the second healthcare provider assistant system 1000.

As shown in Figure 11, the system 100 of the emergency vehicle 1100 is further configured to send the summary 900 as described herein with reference to Figure 9 to another healthcare provider assistant system 1110 and/or to a separate electronic device.

In examples where the healthcare provider assistant system 100 is provided in an emergency vehicle 1100, the whole system 100 may be located within the emergency vehicle 1100, with energy provided by the vehicle or an additional energy storage device. For example, all components and processing of the system 100 may be contained to the vehicle 1100. After the emergency vehicle 1100 reaches its destination, such as a hospital, a data transfer to the second healthcare provider system 1110, which may be located within the hospital, can happen. Alternatively or in addition to this, the data transfer may be to a hospital information system or any IT system capable of receiving and storing the data. This enables the healthcare providers who receive the patient at hospital to review all available information and previous actions within the emergency vehicle 1100 based on for example the summary generated by the system 100 in the vehicle 1100. In examples of a deployment of a system 100 according to examples described herein within the emergency vehicle 1100, the system 100 may be configured to communicate to external systems for example through 5G, Sidelink or Wi-fi or other wireless communication methods.

Figure 12 shows a room 300 of a building in which a healthcare provider assistant system in accordance with an embodiment of the invention. As shown in Figure 12, the room 300 is similar to that shown in Figure 3, in that a patient 320 and a healthcare provider 340 are present in the room and speaking, as represented by patient voice 330 and healthcare provider voice 350. The patient voice 330 and the healthcare provider voice 350 are received by the one or more microphones 120 and provided to a healthcare provider assistant system 100 as described herein. In Figure 12 the system 100 comprises the one or more processors 110 and the display 310 as described herein.

As shown in Figure 12, according to examples the system 100 can additionally comprise one or more speakers 1200. The system is further configured to provide responses to one or more prompts from the set of prompts as text-to-audio output via the one or more speakers.

The system 100 can provide the text-to-audio output via the one or more speakers 1200 whilst the patient 320 and the healthcare provider 340 are conversing, for example whilst the transcription 250 is still being generated and voice data 120 is still being received. The system 100 can therefore intervene in the conversation to provide responses 140 to one or more prompts as text-to-audio output. The system 100 can therefore provide information and/or content via audio whilst in parallel collecting voice data 120 and generating a transcription, providing an improved interaction between humans and the system 100.

According to examples, the system 100 may be configured to translate the transcription 250 into other languages. For translation, a specialized language model for translation of texts might be used. This may either be the trained language model 260, or may be another language model accessible by the system 100. This language model might be further finetuned to texts from the medical domain.

According to examples, the system 100 may be distributed as a cloud-based system. The one or more microphones 130 may be provided in one or more electronic devices such as laptops, smartphones, tablet. For example the patient 320 may be using a first electronic device to record their voice and the healthcare provider 340 may be using a second electronic device to record their voice. Using a cloud-based system 100 according to examples disclosed herein and using the first electronic device and the second electronic device may be used in a remote patient interview. The system 100 according to examples disclosed herein may implement the remote patient interview through a web frontend that is served by the system 100 or through an application deployed on the user devices that handles communication with the software backend with the one or more processors 110 configured to perform the operations disclosed herein on the cloud server.

According to examples disclosed herein, to enable faster inference of the speech recognition model and/or trained language model 260, parallel processing and to enable its deployment on consumer devices such as laptops, tablets, smartphones, etc., quantized versions of the utilized trained language model 260, speech recognition model or other Al-models are used. This means, that weights will not be stored and processed in a float32 or float16 format, but rather in an integer format such int5, int4, int3, int2 or int1.

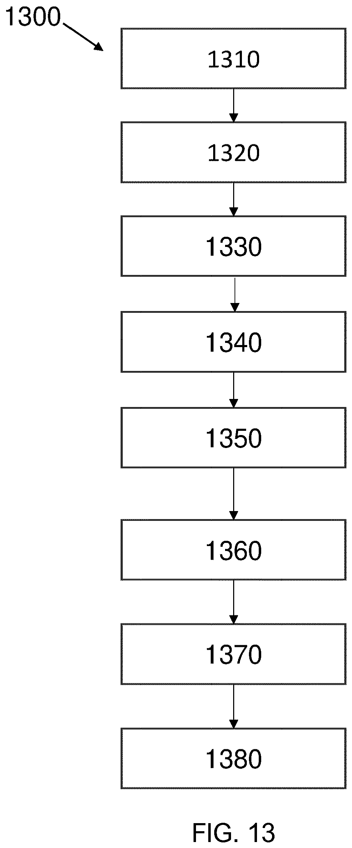

Figure 13 shows a computer-implemented method in accordance with an embodiment of the invention. The computer-implemented method may be performed by a healthcare provider assistant system 100 according to examples and embodiments disclosed herein. The method comprises: receiving 1310 voice data 120 from one or more microphones 130; recognising 1320 a first voice as a healthcare provider voice previously registered; assigning 1330 a second voice as a patient voice; generating 1340 a transcription of the voice data, wherein the transcription identifies words spoken by the healthcare provider voice and words spoken by the patient voice; providing 1350 the transcription and/or the voice data to a trained language model; providing 1360 a set of prompts to the trained language model.

The set of prompts comprise: a first subset of prompts associated with the healthcare provider voice; wherein each prompt comprises one or more tasks for the trained language model to complete, using the transcription and/or voice data; wherein at least one prompt of the first subset of prompts relates to obtaining healthcare data based on words spoken by the healthcare provider voice.

The method further comprises processing 1370 the transcription and/or the voice data and the set of prompts using the trained language model to provide 1380 responses to one or more prompts from the set of prompts.

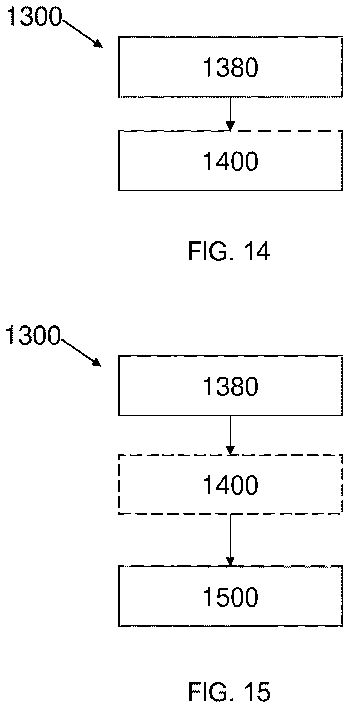

Figure 14 shows part of a computer-implemented method in accordance with an embodiment of the invention. The block 1380 is shown in Figure 14 to show that the other block in Figure 14 occurs after the block 1380 of the method shown in Figure 13.

According to examples, the method shown in Figure 14 additionally comprises storing 1400 the transcription, voice data, response to one or more prompts from the set of prompts on a first memory of a first healthcare provider assistant system arranged to perform the method. The transcription, voice data and responses may therefore be stored for later access on the system 100.

Figure 15 shows part of a computer-implemented method in accordance with an embodiment of the invention. The block 1380 is shown in Figure 15 to show that the other blocks in Figure 15 occurs after the block 1380 of the method shown in Figure 13. Block 1400 from Figure 14 is shown in dotted lines to show that this is an optional part of the method shown in Figure 15. According to examples, the computer-implemented method shown in Figure 15 further comprises: transferring 1500 the transcription, voice data, responses from the first healthcare provider assistant system to a second healthcare provider assistant system arranged to perform the method.

As described herein with reference to Figures 9A, 9B and 10, transcription, voice data, responses can be transferred between different healthcare provider assistant systems which means that different healthcare sessions on a particular healthcare provider assistant system can use information and content previously obtained from another healthcare provider assistant system, which can enhance the overall healthcare experience of the patient.

According to examples, the first healthcare provider assistant system can be provided in an emergency vehicle, and the second healthcare provider assistant system can be provided in a room of a building. Beneficially therefore information, such as events that took place in the emergency vehicle and information regarding the patient's condition or illness, can be immediately accessible to subsequent healthcare provider assistant systems and the users of those systems, which results in a more efficient healthcare process.

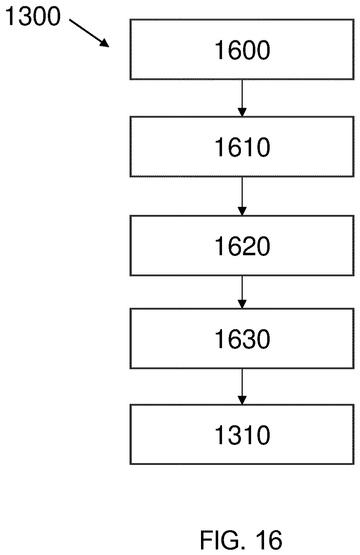

Figure 16 shows part of a computer-implemented method in accordance with an embodiment of the invention. The block 1310 is shown in Figure 16 to show that the other blocks in Figure 15 occur before the block 1310 of the method shown in Figure 13

According to examples and as shown in Figure 16, prior to receiving the voice data from the one or more microphones, the method can additionally comprise: receiving 1600 a request to register the first voice as the healthcare provider voice; obtaining 1610 a sample voice recording of the first voice; calculating 1620 voice embeddings to determine features of the first voice; storing 1630 the voice embeddings as being associated with the first voice; using 1640 the voice embeddings to recognise the first voice as the healthcare provider voice in the voice data. As discussed herein with reference to Figure 7, users wishing to be registered as healthcare provider voices can register with the system so that during a session the system 100 will automatically recognise their voice and use it for the purposes of the prompts to be carried out and for the generation of the transcription, whilst assigning unknown voices during a session as patient voices.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

## Claims

1. A healthcare provider assistant system comprising:
one or more processors collectively configured to:
receive voice data from one or more microphones;
recognise a first voice as a healthcare provider voice previously registered with the healthcare provider assistant system;
assign a second voice as a patient voice;
generate a transcription of the voice data, wherein the transcription identifies words spoken by the healthcare provider voice and words spoken by the patient voice;
provide the transcription and/or the voice data to a trained language model;
provide a set of prompts to the trained language model;
wherein the set of prompts comprise:
a first subset of prompts associated with the healthcare provider voice;
wherein each prompt comprises one or more tasks for the trained language model to complete, using the transcription and/or voice data;
wherein at least one prompt of the first subset of prompts relates to obtaining healthcare data based on words spoken by the healthcare provider voice;
wherein the trained language model is arranged to process the transcription and/or the voice data and the set of prompts to provide responses to one or more prompts from the set of prompts.

2. A healthcare provider assistant system as claimed in claim 1, wherein the set of prompts additionally comprise a second subset of prompts associated with the patient voice, wherein optionally at least one prompt of the second subset of prompts relates to obtaining healthcare data based on words spoken by the patient voice.

3. A healthcare provider assistant system as claimed in any preceding claim, additionally comprising a display, wherein the display is configured to display at least one of: information and generated content resulting from responses to one or more prompts from the set of prompts, and optionally wherein the healthcare provider assistant system is further configured to display the information relating to the responses to one or more prompts on the display whilst receiving additional voice data from the one or more microphones and continuing to generate the transcription of the voice data.

4. A healthcare provider assistant system as claimed in any preceding claim, further configured to generate the transcription and continue updating the transcription as additional voice data from the one or more microphones is received, whilst obtaining at least one of: information and generated content resulting from responses to one or more prompts from the set of prompts.

5. A healthcare provider assistant system as claimed in any preceding claim, wherein the one or more processors further comprise an orchestrator module, the orchestrator module configured to:
select the prompts which form the set of prompts which are provided to the trained language model for processing;
control the provision of the transcription and/or the voice data to the trained language model;
process the responses to one or more prompts from the set of prompts;
determine if one or more of the responses are for further processing as inputs to the trained language model in a subsequent inference of the trained language model and/or as inputs to a software module;
control one or more subsequent inferences of the trained language model to cause further processing of the responses and/or controlling the software module to process the one or more responses.

6. A healthcare provider assistant system as claimed in any preceding claim, wherein one or more of the first subset of prompts relate to asking the patient for consent to store the voice data and/or generate the transcription, wherein the system is configured to determine whether the patient provides their consent based on the voice data relating to the patient voice;
wherein if the system determines that the patient does not provide their consent, the healthcare provider assistant system prohibits the generating of the transcription of the voice data, storage of the voice data and the providing of the transcription and/or providing the voice data to the trained language model.

7. A healthcare provider assistant system as claimed in any preceding claim, wherein the healthcare provider system is implemented on an electronic device; wherein the one or more of the first subset of prompts relates to asking the patient for consent for the voice data and/or transcript to be processed non-locally; wherein the one or more processors are configured to determine whether the patient provides their consent for the voice data and/or the transcription to be processed non-locally;
wherein if the patient provides their consent then the healthcare provider assistant system is permitted to use an external trained language model not stored on the one electronic device as the trained language model;
wherein if the patient does not provide their consent then the healthcare provider assistant system is restricted to using a language model stored on the electronic device.

8. A healthcare provider assistant system as claimed in any preceding claim, further configured to:
receive a request to register the first voice as the healthcare provider voice;
obtain a sample voice recording of the first voice;
calculate voice embeddings to determine features of the first voice;
store the voice embeddings as being associated with the first voice;
use the voice embeddings to recognise the first voice as the healthcare provider voice in the voice data.

9. A healthcare provider assistant system as claimed in any preceding claim, wherein one or more of:
the healthcare data comprises data for providing a prescription recommendation, wherein the trained language model is configured to access one or more data stores which store prescription information to provide the prescription recommendation;
wherein the healthcare data comprises data for providing a referral recommendation for the patient, wherein the trained language model is configured to access one or more data stores which store referral information to provide the referral recommendation, wherein the healthcare data comprises treatment guidelines for the patient;
wherein the trained language model is configured to access one or more data stores which store treatment information to provide the treatment guidelines; wherein the healthcare data comprises one or more of: medical sensor data from at least one medical sensor;
wherein the healthcare data comprises diagnosis data; and
wherein the healthcare data comprises electronic medical records of the patient.

10. A healthcare provider assistant system as claimed in any preceding claim, further configured to extract key information from the voice data and generate a summary comprising the key information, wherein the key information comprises one or more of:
the patient's name;
the reason the patient consulted the healthcare provider;
symptoms described by the patient;
pre-existing conditions of the patient;
medications which the patient already takes;
examinations conducted by the healthcare provider;
diagnosis which the healthcare provider gives;
prescriptions which the healthcare providers gives;
referrals for further examination the healthcare provider gives,
wherein optionally, the healthcare provider assistant system is further configured to send the summary of the transcription to another healthcare provider assistant system and/or to a separate electronic device.

11. A healthcare provider assistant system as claimed in any preceding claim, further configured to generate a formatted document based on the voice data and/or the transcription, comprising one or more of: a diagnosis, a summary of session, a recommended treatment, a recommended prescription; and/or
wherein the system additionally comprises one or more speakers, wherein the system is further configured to provide responses to one or more prompts from the set of prompts as text-to-audio output via the one or more speakers.

12. An emergency vehicle comprising the healthcare provider assistant system of any preceding claim.

13. A computer-implemented method for assisting a healthcare provider, the method comprising:
receiving voice data from one or more microphones;
recognising a first voice as a healthcare provider voice previously registered;
assigning a second voice as a patient voice;
generating a transcription of the voice data, wherein the transcription identifies words spoken by the healthcare provider voice and words spoken by the patient voice;
providing the transcription and/or the voice data to a trained language model;
providing a set of prompts to the trained language model;
wherein the set of prompts comprise:
a first subset of prompts associated with the healthcare provider voice;
wherein each prompt comprises one or more tasks for the trained language model to complete, using the transcription and/or voice data;
wherein at least one prompt of the first subset of prompts relates to obtaining healthcare data based on words spoken by the healthcare provider voice;
processing the transcription and/or the voice data and the set of prompts using the trained language model to provide responses to one or more prompts from the set of prompts.

14. The computer-implemented method of claim 13, further comprising:
storing the transcription, voice data, response to one or more prompts from the set of prompts on a first memory of a first healthcare provider assistant system arranged to perform the method; or
transferring the transcription, voice data, responses from the first healthcare provider assistant system to a second healthcare provider assistant system arranged to perform the method.

15. The computer-implemented method of claim 14 when transferring the transcription, void data, responses from the first healthcare provider assistant system to a second healthcare provider assistant system arranged to perform the method, wherein the first healthcare provider assistant system is provided in an emergency vehicle, and the second healthcare provider assistant system is provided in a room of a building.
